(19) Europäisches Patentamt
European Patent Office
Office européen des brevets

(11) Publication number: **0 124 618**
**A1**

(12) # EUROPEAN PATENT APPLICATION

published in accordance with Art. 158(3) EPC

(21) Application number: **83903415.4**

(22) Date of filing: **02.11.83**

Data of the international application taken as a basis:

(86) International application number:
**PCT/JP83/00394**

(87) International publication number:
**WO84/01950 (24.05.84 84/13)**

(51) Int. Cl.³: **C 07 D 501/36**
A 61 K 31/545

(30) Priority: **10.11.82 JP 197171/82**

(43) Date of publication of application:
**14.11.84 Bulletin 84/46**

(84) Designated Contracting States:
**CH DE FR GB LI**

(71) Applicant: **Kyoto Pharmaceutical Industries, Ltd.**
**38, Nishinokyo Tsukinowa-cho Nakakyo-ku**
**Kyoto-shi Kyoto 604(JP)**

(72) Inventor: **KAKEYA, Nobuharu**
**10-16, Takadai 3-chome Nagaokakyo-shi**
**Kyoto 617(JP)**

(72) Inventor: **NISHIZAWA, Susumu**
**1, Yamaden Shimomisu Yokooji**
**Fushimi-ku Kyoto-shi, Kyoto 612(JP)**

(72) Inventor: **TAMAKI, Satoshi**
**1665-24 Kojimacho, Moriyama-shi**
**Shiga 524(JP)**

(72) Inventor: **KITAO, Kazuhiko**
**12, Santan Osa-cho Matugasaki Sakyo-ku**
**Kyoto-shi Kyoto 606(JP)**

(74) Representative: **von Kreisler, Alek et al,**
**Patentanwälte Von Kreisler-Schönwald-Fues-Keller**
**Selting-Werner Deichmannhaus am Hauptbahnhof**
**D-5000 Köln 1(DE)**

(54) **CEPHALOSPORIN DERIVATIVES, PROCESS FOR THEIR PREPARATION, AND AGENTS FOR PROPHYLAXIS OR TREATMENT OF BACTERIA-INDUCED DISEASES.**

(57) Cephalosporin derivatives represented by formula (I)

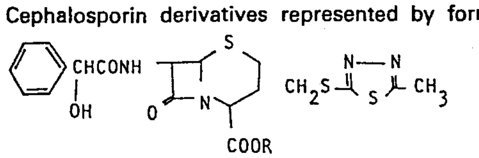

I

(wherein R represents $-CH_2-OCOR_{a}1$ (wherein $R_a1$ represents an alkyl group),

–CH–OCORb'
|
$CH_3$

(wherein $R_b1$ represents an alkyl group),

–CH–OCOORc'
|
$CH_3$

(wherein $R_c1$ represents an alkyl group),

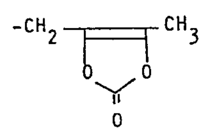

or phthalidyl group), are rapidly absorbed by blood when administered orally to exhibit an excellent antibacterial effect, thus being useful as agents to be applied orally for prophylaxis or treatment of bacteria-induced diseases.

EP 0 124 618 A1

SPECIFICATION

TITLE MODIFIED
see front page

Cephalosporin derivatives, methods for production thereof and compositions for the prevention and treatment of bacterial infection

TECHNICAL FIELD

This invention relates to cephalosporin derivatives.

BACKGROUND ART

Cephalosporins are in general poorly absorbed through the digestive tract and, thus, administered with an injection.

For example, the cephalosporin of the formula:

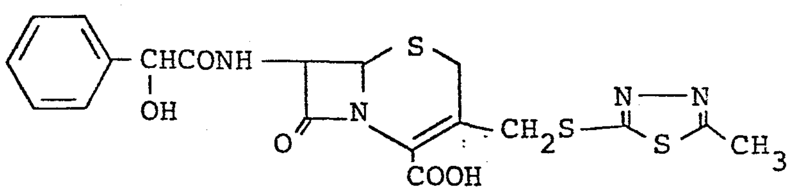

(II)

has excellent antibacterial activities, but due to its poor absorbability through the digestive tract, it is administered with an injection.

As a result of extensive research to obtain orally administrable cephalosporin preparations, the present inventors have completed this invention, on the basis of the findings that the cephalosporin derivatives (I) mentioned below are of good absorbability through the digestive tract and are rapidly

after absorption, hydrolyzed in vivo under the action of enzymes, whereby the ester moiety of the substituent in the 4-position of the cephem ring structure is hydrolyzed and cephalosporin derivatives (I) are thereby converted to the corresponding unesterified species, namely, cephalosporins of the above formula (II) (hereinafter referred to as unesterfied species (II)), that is, that oral administration of cephalosporin derivatives (I) results in high blood levels over prolonged period of the unesterified species that have excellent antibacterial activities.

## DISCLOSURE OF INVENTION

This invention relates to cephalosporin derivatives of the general formula:

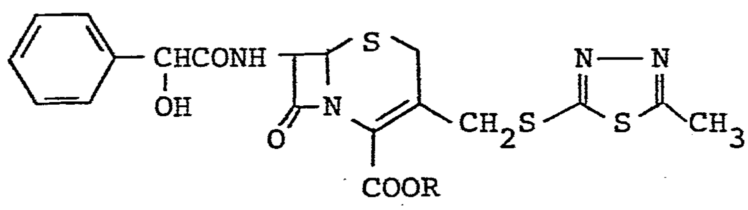

(I)

wherein R represents $-CH_2OCOR_a^1$ (wherein $R_a^1$ represents an alkyl group), $-CH(CH_3)-OCOR_b^1$ (wherein $R_b^1$ represents an alkyl group, $-CH(CH_3)-OCOOR_c^1$ (wherein $R_c^1$ represents an alkyl group), 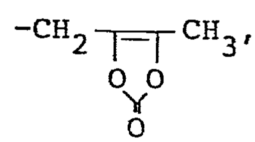 or phthalidyl group.

In this specification, an alkyl group means concept including both a straight one and branched one, preferably

having 1 to 4 carbon atoms such as methyl, ethyl, propyl, iso-propyl, butyl, iso-butyl, t-butyl and the like.

Cephalosporin derivatives (I) can be prepared, for instance, by the following method. Namely, they can be prepared by reacting an unesterified species (II) with a compound of the general formula (III)

$$R - OH \qquad (III)$$

wherein R is as defined above.

Unesterified species (II) and compound (III) may be subjected to the reaction directly as they are, but preferably it is convenient that one of the unesterified species (II) and compound (III) is converted or both of them are converted to a reactive derivative thereof and then the reaction is carried out. The reactive derivatives of unesterified species (II) mean those on the carboxyl group and such reactive derivatives are exemplified by those which are generally used for esterification. Namely, as such reactive derivatives, there may be used such mineral salts as alkali metal salts (e.g. sodium salt and potassium salt) and such organic salts as organic amine salts (e.g. triethylamine salt). As examples of the reactive derivatives of compound (III), there may be mentioned alkyl- or arylsulfonic acid esters such as methanesulfonic acid ester and p-toluenesulfonic acid ester, diesters of sulfuric acid and sulfurous acid, alkyl- or arylchloroformate (e.g. ethylchloroformate, phenylchloroformate), alkyloxy- or aryloxysulfenic acid ester, alkyloxy- or aryloxysulfonic aicd ester, and halides which are derived by substituting hydroxy

group of compound (III) by a halogen such as chlorine, bromine, iodine. It is preferred to react, for example, a salt of the unesterified species (II) with halide of compound (III) at room temperature or, if necessary, under cooling for preventing formation of byproduct of $\Delta^2$-isomer.

The present reaction can be carried out readily in the presence of a solvent which does not interfere with the reaction, such as dimethylformamide dimethylacetamide, hexamethylphosphorictriamide, acetone, acetonitrile and the like.

Further, cephalosporin derivatives (I) can be prepared by reacting a compound of the general formula:

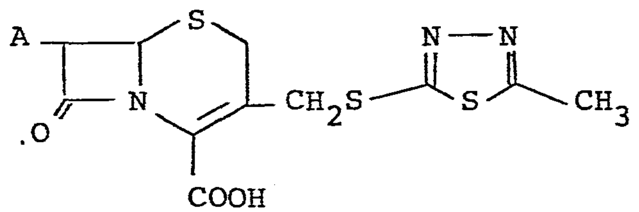

(IV)

wherein A represents amino group or a protected amino group, with a compound (III) in the same manner as the above-mentioned esterification, followed, in the case where A is a protected amino group, by removal of the protective group of the obtained esterified compound by a conventional method, and further acylating the amino group in the 7-position of the derived esterified compound of the general formula:

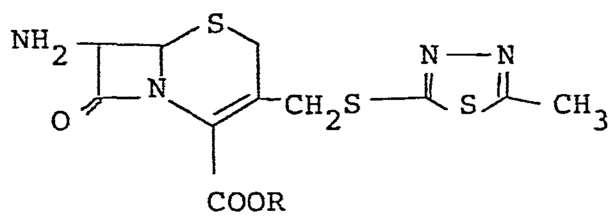

(V)

wherein R has the same meaning as above, with a compound of the formula:

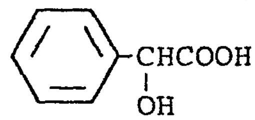

(VI)

Referring to the above general formula (IV), in the case where A is a protected amino, as the protective group, there may be used any of per se known ones in the cephalosporin field. When the protective group is amino-containing group, it is preferable that the amino group in the amino-containing group is, in carrying out the reaction, protected. Such protective groups for the amino group, may be suitably selected from among per se known protective groups for amino group such as benzylcarbonyl, 2-thienylacetyl, 5-amino-5-carbaoxyvaleryl, t-butoxycarbonyl, benzyloxycarbonyl, trityl, phthalimide group. The hydroxy group of compound (VI) may be, in carrying out the reaction, protected. As examples of the protective groups therefor, there may be mentioned formyl group, silyl group, dichloroacetyl group and the like. Compound (VI) is used in the free carboxylic acid form or in the form of a reactive derivative thereof. Thus, it is submitted to the acylation reaction in the form of a free acid or reactive derivative, for example, a sodium, potassium, calcium, trimethylamine or pyridine salt, an acid halide, an acid anhydride, a mixed acid anhydride, a cyclic carboxy anhydride, a reactive amide or ester, and the like.

In cases where compound (VI) is used in the free acid

or salt form, the use of an adequate condensing agent is preferable and usable condensing agents are, for example, such dehydrating agents as N,N'-disubstituted carbodiimides (e.g. N,N-dicyclohexylcarbodiimide) and azolide compounds (e.g. N,N'-carbonylimidazole, N,N'-thionyldiimidazole). When such a condensing agent is used, the reaction presumably proceed via a reactive derivative of the carboxylic acid.

The cephalosporin derivatives (I) can be isolated and purified by a *per se* known method.

By diluting the thus-produced cephalosporin derivatives (I) with a pharmaceutical carrier or excipient by a *per se* known means, the orally administrable therapeutic preparations for the prevention and treatment of bacterially infections diseases can be prepared. The dilution is carried out by a *per se* known means such as mixing or the like. As examples of the carrier or excipient, there may be mentioned starch, lactose, sugar, calcium carbonate and calcium phosphate.

If desired, other additives may be further added to said orally administrable pharmaceutical preparations for the prevention and treatment of bacterial infection. Preferred additives are, for instance, binders (e.g. polyvinylpyrrolidone, starch, gum arabic, carboxymethylcellulose, hydroxypropylcellulose, crystalline cellulose), lubricant (e.g. magnesium stearate, talc) and disintegrators (e.g. calcium carboxymethylcellulose, talc and the like). After mixing the ingredients, the mixture can be formed into dosage forms suited for oral administration, such as capsules, powder, fine-granules,

granules, and dry syrup, by per se known means.

Cephalosporin derivatives (I) according to the invention can be rapidly absorbed through the digestive tract and are rapidly after absorption, hydrolyzed in vivo under the action of enzymes to be converted into the corresponding unesterified species or salts thereof.

Such unesterified species (II) and salts thereof have excellent antibacterial activities. Thus, they exhibit excellent activity against such gram-positive bacteria as Staphylococcus aureus and such gram-negative bacteria as Escherichia coli, Klebsiella pneumoniae, Proteus vulgaris, Proteus mirabillis, Proteus morganu. In addition, the unesterified species have very low toxicity.

Therefore, cephalosporin derivatives (I) according to the present invention can be used as therapeutic agents, for example, for bacteria caused diseases (e.g. suppurative diseases, respiratory tract infection, biliary tract infection, urinary tract infection) in warm-blooded aminals including humans (e.g. dog, cat, cattle, horse, rat, mouse).

The dose of cephalosporin derivatives (I) varies depending upon, host to be administered to , severity of symtom and other factors. In treating suppurative diseases in human adults, for instance, cephalosporin derivatives (I) are administered orally at a dose of about 1-20 mg (as the corresponding unesterified species) per kg body weight three or four times a day.

Furthermore, cephalosporin derivatives according to

this invention are also useful as intermediates for synthesizing such cephalosporin derivatives, for example, as disclosed in the specification of PCT/JP82/00437.

Example 1

Preparation of acetoxymethyl 7-[D-mandelamido]-3-[(5-methyl-1,3,4-thiadiazol-2-yl)thiomethyl]-3-cephem-4-carboxylate:

In 45 ml of dimethylformamide was dessolved 1.5 g of potassium salt of 7-[D-mandelamido]-3-[(5-methyl-1,3,4-thiadiazol-2-yl)thiomethyl]-3-cephem-4-carboxylic acid and the solution was cooled to -20°C, whereto 612 mg of iodomethylacetate was added under stirring. The mixture was allowed to react at the same temperature for 30 mixture and, following addition of 200 mg of ethyl acetate thereto, washed with ice water (100 ml x 3) and then with a saturated aqueous sodium chloride solution (100 ml x 2), and dried over anhydrous sodium sulfate. The solution was concentrated and crystalized from petroleum ether to give 1.01 g of the title compound as pale yellow powder.

IR (nujol, $cm^{-1}$): 3350, 1780, 1740, 1680

NMR ($CDCl_3$, δ value): 2.13 (s, 3H, $-OCOCH_3$), 2.71 (s, 3H, thiadiazole, $-CH_3$), 3.67 (br. s, 2H, $-H_2$ at posiiton 2), 3.81 (s, 1H, -OH), 4.13, 4.20 (d, d, 2H, J=14Hz, $-CH_2$-S- at position 3), 4.95 (d, 1H, J=5Hz, -H at position 6), 5.13 (s, 1H, -CH-OH), 5.74 (d x d, 1H, J=5Hz, 9Hz, -H at position 7), 5.85, 5.96 (d, d, 2H, J=7Hz, $-CO_2CH_2-$), 7.14 (d, 1H, J=9Hz, -CONH-), 7.38 (s, 5H, phenyl)

Example 2

Preparation of 1'-acetoxyethyl 7-[D-mandelamido]-3-[(5-methyl-1,3,4-thiadiazol-2-yl)thiomethyl]-3-cephem-4-carboxylate:

In 80 ml of dimehtylacetamide was dissolved 4 g of potassium salt of 7-[D-mandelamido]-3-[(5-methyl-1,3,4-thiadiazol-2-yl)thiomethyl]-3-cephem-4-carboxylic acid, and the solution was cooled to -20°C, whereto 2.4 g of 1'-iodoethylacetate was added. The mixture was stirred at the same temperature for 1 hour. After 500 ml of ethyl acetate was added thereto, the mixture was washed with water (200 ml x 3) and then with a saturated aqueous sodium chloride solution (100 ml x 2) and dried over anhydrous sodium sulfate. The solution was concentrated, whereto 100 ml of isopropyl ether was added, to give 3.6 g of the title compound as crystalline white powder.

IR (nujol, $cm^{-1}$): 3350, 1780, 1740, 1680

NMR ($(CD_3)_2SO$, δ value): 1.48 (d, 1.5H, J=6Hz, $-\overset{|}{C}H\underline{CH_3}$), 1.50 (d, 1.5H, J=6Hz, $-\overset{|}{C}H\underline{CH_3}$), 2.02 (s, 1.5H, $-OCOCH_3$), 2.06 (s, 1.5H, $-OCOCH_3$), 2.70 (s, 3H, thiadiazole-$CH_3$), 3.68 (br. s, 2H, $-H_2$ at position 2), 4.12, 4.52 (d, d, 1H, J=14HZ, $CH_2S$- at position 3), 4.14, 4.54 (d, d, 1H, J=14Hz, $-CH_2S$- at position 3), 5.09 (m, 2H, $-\underline{\overset{|}{C}H}OH$, -H at position 6), 5.70 (m, 1H, -H at potition 7), 6.10 (d, 1H, J=6Hz, $-\overset{|}{C}H\underline{OH}$), 6.90 (q, 0.5H, J=6Hz, $-\underline{\overset{|}{C}H}CH_3$), 6.98 (q, 0.5H, J=6Hz, $-\underline{\overset{|}{C}H}CH_3$), 7.43(m, 5H, phenyl), 8.70 (d, 1H, J=9Hz, -CONH-)

Example 3

Preparation of 1'-isobutyryloxyethyl 7-[D-mandelamido]-3-[(5-methyl-1,3,4-thiadiazol-2-yl)thiomethyl]-3-cephem-4-carboxylate:

In 60 ml of dimehtylacetamide was dissolved 3 g of potassium salt of 7-[D-mandelamido]-3-[(5-methyl -1,3,4-thiadiazol-2-yl)thiomethyl-3-cephem-4-carboxylic acid, whereto potassium acetate was added, and the mixture was cooled to -20°C. After 2.2 g of 1'-iodoethylisobutylate was added, the mixture was stirred at the same temperature for 30 minutes. Thereto, 200 ml of ethylacetate was added and the mixture was washed with cold water (100 ml x 3) and then with a saturated aqueous sodium chloride solution (50 ml x 2) and dried over anhydrous sodium sulfate. The solution was concentrated, followed by addition of 50 ml of isopropyl ether to give 3.5 g of the title compound as crystalline white powder.

IR (nujol, $cm^{-1}$): 3350, 1780, 1740, 1680

NMR ($(CD_3)_2CO$, δ value): 1.15 (d, 6H, J=7Hz, $-CH(\underline{CH_3})_2$), 1.53 (d, 1.5H, J=6Hz, $-CH\underline{CH_3}$), 1.57 (d, 1.5H, J=6Hz, $-CH\underline{CH_3}$), 2.2-3.0 (m, 1H, $-\underline{CH}(CH_3)_2$), 2.70 (s, 3H, thiadiazole-$CH_3$), 3.68 (br. s, 2H, $-H_2$ at position 2), 4.13, 4.45 (d, d, 1H, J=14Hz, $-CH_2S-$ at position 3), 4.20, 2.60 (d, d, 1H, J=14Hz, $-CH_2S-$ at position 3), 5.10 (m, 2H, $-\underline{CH}OH$, -H at position 6), 5.72 (m, 1H, -H at position 7), 6.07 (d, 1H, J=6Hz, $-CH\underline{OH}$), 6.91 (q, 0.5H, J=6Hz, $-\underline{CH}CH_3$), 6.99 (q, 0.5H, J=6Hz, $-\underline{CH}CH_3$), 7.38 (m, 5H, phenyl), 7.98 (d, 1H, J=9Hz, -CONH-)

Example 4

Preparation of (5-methyl-1,3-dioxolen-2-on-4-yl)methyl 7-[D-mandelamido]-3-[(5-methyl-1,3,4-thiadiazol-2-yl)thiomethyl]-3-cephem-4-carboxylate:

In 40 ml of dimethylacetamide was dissolved 2.2 g of potassium salt of 7-[D-mandelamido]-3-[(5-methyl-1,3,4-thiadiazol-2-yl)thiomethyl]-3-cephem-4-carboxylic acid and 1.2 g of 4-bromomethyl-5-methyl-1,3-dioxolen-2-one was added thereto at -20°C. The mixture was stirred at the same temperature for 1 hour. After completion of the reaction, 400 ml of ethylacetate was added, and the mixture was washed with water (200 ml x 3) and then with a saturated aqueous sodium chloride solution (100 ml x 2), and was dried over anhydrous sodium sulfate. Following concentration of the solution, the obtained residue was subjected to chromatography on 40 g of silica gel (Kieselge 60) using benzene-ethylacetate (1:1) as the eluant to give 1.2 g of the title compound.

IR (nujor, $cm^{-1}$): 3350, 1820, 1780, 1730, 1680

NMR ($(CD_3)_2CO$, δ value): 2.22 (s, 3H, dioxolene-$CH_3$), 2.70 (s, 3H, thiadiazole-$CH_3$), 3.59, 3.97 (d, d, 2H, J=18Hz, $-H_2$ at position 2), 4.07, 4.87 (d, d, 2H, J=14Hz, $-CH_2S-$ at position 3), 5.21 (d, 1H, J=6Hz, -CHOH), 5.15 (s, 2H, $-COOCH_2-$), 5.41 (d, 1H, J=6Hz, -CHOH), 5.12 (d, 1H, J=4Hz, -H at position 6), 5.81 (d x d, 1H, J=9Hz, 4.8Hz, -H at position 7), 7.42 (m, 5H, phenyl), 7.99 (d, 1H, J=9Hz, -CONH-)

## Example 5

Preparation of phthalidyl 7-[D-mandelamido]-3-[(5-methyl -1,3,4-thiadiazol-2-yl)thiomethyl]-3-cephem-4-carboxylate:

According to the same procedure as Example 4, using 1.1 g of 3-bromophtanlide instead of 4-bromomethyl-5-methyl-1,3-dioxolen-2-one, 1.0 g of the title compound was obtained as pale yellow powder.

IR (nujol, $cm^{-1}$): 3350, 1780, 1680

NMR ($CDCl_3$, δ value): 2.70 (s, 3H, thiadiazole-$CH_3$), 3.72 (br. s, 2H, $-H_2$ at position 2), 4.2, 4.85 (d, d, 2H, J=14Hz, $-CH_2S-$ at position 3), 5.15 (d, 1H, J=7Hz, -H at position 6), 5.38 (br. s, 1H, -CH-OH), 5.65 (m, 1H, -H at position 7), 7.34 (m, 5H, phenyl), 7.52 (d, 1H, -COOCH-), 7.8 (m, 4H, phenyl), 8.64 (br. d, 2H, -CONH-)

## Example 6

Preparation of pivaloyloxymethyl 7-[D-mandelamido]-3-[(5-methyl-1,3,4-thiadiazol-2-yl)thiomethyl]-3-cephem-4-carboxylate:

According to the same procedure as Example 1 using 750 mg of iodomethylpivalate instead of iodomethyl-acetate, 1.2 g of the title compound was obtained as pale yellow powdery substance.

IR (nujol, $cm^{-1}$): 3350, 1780, 1740, 1680

NMR ($(CD_3)_2SO$, δ value): 1.17 (s, 9H, $-C(CH_3)_3$), 2.70 (s, 3H, thiadiazole-$CH_3$), 3.70 (br. s, 2H, $-H_2$ at

position 2), 4.14, 4.58 (d, d, 2H, J=14Hz, $-CH_2S-$ at position 3), 5.10 (m, 2H, -CHOH, -H at position 6), 5.83 (m, 3H, $-COOCH_2-$, -H at position 7), 6.10 (d, 1H, J=6Hz, -CHOH), 7.40 (m, 5H, phenyl), 8.75 (d, 1H, J=9Hz, -CONH-)

## Example 7

Preparation of 1'-propionyloxyehtyl 7-[D-mandelamido]-3-[(5-methyl-1,3,4-thiadiazol-2-yl)thiomethyl]-3-cephem-4-carboxylate:

According to the same procedure as Example 2, using 2.6 g of 1'-iodoethylpropionate instead of 1'-iodoethylacetate, 3.7 g of the title compound was obtained as pale yellow powder.

IR (nujol, $cm^{-1}$): 3350, 1780, 1740, 1680

NMR ($(CD_3)_2SO$, δ value): 1.10 (t, 3H, J=6Hz, $-CH_2CH_3$), 1.50 (d, 1.5H, J=6Hz, $-CHCH_3$), 1.52 (d, 1.5H, J=6Hz, $-CHCH_3$), 2.35 (q, 2H, J=6Hz, $-CH_2CH_3$), 2.70 (s, 3H, thiadiazole-$CH_3$), 3.67 (br. s, 2H, $-H_2$ at position 2), 4.15, 4.50 (d, d, 2H, J=14Hz, $-CH_2S-$ at position 3), 5.10 (m, 2H, -CHOH, -H at position 6), 5.71 (m, 1H, -H at position 7), 6.10 (d, 1H, J=6Hz, -CHOH), 6.88 (q, 0.5H. J=6Hz, $-CHCH_3$), 6.96 (q, 0.5H, J=6Hz, $-CHCH_3$), 7.38 (m, 5H, phenyl), 8.72 (d, 1H, J=9Hz, -CONH-)

## Example 8

Preparation of 1'-ethoxycarbonyloxyethyl 7-[D-mandelamido]-3-[(5-methyl-1,3,4-thiadiazol-2-yl)thiomethyl]-3-cephem-4-carboxylate:

According to the same procedure as Example 2, using 2.6 g of 1'-iododiethylcarbonate instead of 1'-iodoethylacetate, 3.5 g of the title compound was obtained as pale yellow powdery substance.

IR (nujol, $cm^{-1}$): 3350, 1780, 1760, 1680

NMR ($(CD_3)_2SO$, δ value): 1.21 (t, 1.5H, J=7Hz, $-OCH_2\underline{CH_3}$), 1.23 (t, 1.5H, J=7Hz, $-OCH_2\underline{CH_3}$), 1.51 (d, 1.5H, J=6Hz, $-\overset{|}{C}H\underline{CH_3}$), 1.53 (d, 1.5H, J=6Hz, $-\overset{|}{C}H\underline{CH_3}$), 2.68 (s, 3H, thiadiazole-$CH_3$), 3.70 (br. s, 2H, $-H_2$ at position 2), 3.95-4.70 (m, 4H, $O\underline{CH_2}CH_3$, $-CH_2S-$ at position 3), 5.07 (m, 2H, $-\underline{\overset{|}{C}H}OH$, -H at position 6), 5.75 (m, 1H, -H at position 7), 6.09 (d, 0.5H, J=6Hz, $-\overset{|}{C}H\underline{OH}$), 6.11 (d, 0.5H, J=6Hz, $-\overset{|}{C}H\underline{OH}$), 6.80 (q, 0.5H, J=6Hz, $-\underline{\overset{|}{C}H}CH_3$), 6.87 (q, 0.5H, J=6Hz, $-\underline{\overset{|}{C}H}CH_3$), 7.15-7.60 (m, 5H, phenyl), 8.69 (d, 1H, J=9Hz, -CONH-)

## Example 9

Preparation of 1'-pivaloyloxyethyl 7-[D-mandelamido]-3-[(5-methyl-1,3,4-thiadiazol-2-yl)thiomethyl]-3-cephem-4-carboxylate:

According to the same procefure as Example 2, employing 2.4 g of 1'-iodoethylpibalate instead of 1'-iodoethylacetate, 3.6 g of the title compound was obtained as pale yellow powder.

IR (nujol, $cm^{-1}$): 3345, 1780, 1740, 1680

NMR ($CDCl_3$, δ value): 1.21 (s, 9H, $-C(CH_3)_3$), 1.54 (d, 1.5H, J=6Hz, $-\overset{|}{C}H\underline{CH_3}$), 1.58 (d, 1.5H, J=6Hz, $-\overset{|}{C}H\underline{CH_3}$), 2.70 (s, 3H, thiadiazole-$CH_3$), 3.66 (br.

s, 2H, $-H_2$ at position 2), 3.88-4.56 (m, 3H, $-CH_2S-$ at position 3, -CHOH), 4.95 (d, 1H, J=5Hz, -H at position 6), 5.14 (s, 1H, -CHOH), 5.63-5.97 (m, 1H, -H at position 7), 7.05 (m, 1H, $-CHCH_3$), 7.37 (br. 6H, phenyl, -CONH-)

Pharmaceutical Recipe Example 1

In accordance with the composition given below an aqueous solution of hydroxypropylcellulose was added to and mixed with a premix prepared from the compound in Example 1, tartaric acid and starch, and then the mixture was subject to screening through drying and pulverizing. A premix prepared from a portion of starch and magnesium stearate was added to and mixed with said screened powder. The resulting mixture is tableted.

| | |
|---|---|
| Compound in Example 2 | 62.5 mg (potency) |
| Polyvinylpyrrolidone | 100 mg |
| Magnesium stearate | 2 mg |
| Hydroxypropylcellulose | 2 mg |
| Starch in an amount to make the total weight | 200 mg |

Pharmaceutical Recipe Example 2

Tablets each having the composition shown below are prepared according to the manner in Pharmaceutical Recipe Example 1.

| | |
|---|---|
| Compound in Example 2 | 62.5 mg (potency) |
| Magnesium stearate | 2 mg |
| Hydroxypropylcellulose | 2 mg |
| Starch in an amout to make the total weight | 150 mg |

Pharmaceutical Recipe Example 3

Tablets each having the composition shown below are prepared by the manner in Pharmaceutical Recipe Example 1.

| | |
|---|---|
| Compound in Example 3 | 62.5 mg (potency) |
| Polyvinylpyrrolidone | 50 mg |
| Magnesium stearate | 2 mg |
| Hydroxypropylcellulose | 2 mg |
| Starch in an amount to make the total weight | 200 mg |

Pharmaceutical Recipe Example 4

In accordance with the composition shown below, starch, compound prepared according to Example 2 and tartaric acid are added to and mixed with a premix prepared from a portion of starch and magnesium stearate, and capsules are filled with the thus-obtained mixture in a conventional manner.

| | |
|---|---|
| Compound in Example 2 | 62.5 mg (potency) |
| Polyvinylpyrrolidone | 50 mg |
| Magnesium stearate | 1 mg |
| Starch in an amount to make the total weight | 150 mg |

Pharmaceutical Recipe Example 5

A dry syrop is prepared according to the following composition.

| | |
|---|---|
| Hydrochloric acid salt of compound in Example 3 | 62.5 mg (potency) |
| Polyvinylpyrrolidone | 100 mg |
| Glucose | 200 mg |
| CMC-Na | 20 mg |

## Acute Toxicity Test

The cephalosporin derivatives of the present invention were orally administered to mice. The acute toxicity data are as follows:

Host for administration:

Male mice (ICR, 5-week-old), n=3

Method of administration:

The cephalosporin derivatives as prepared in the above Examples were orally administered in the form of suspension in 1% methylcellulose solution to mice at doses of 0.5 to 5.0 g/kg.

Results:

| Compound | $LD_{50}$ (g/kg) |
|---|---|
| Example 2 | >5.0 |
| Example 4 | >5.0 |

## Oral Administration Test

The cephalosporin derivatives of the present invention were orally administered to humans. The urinary recovery rates found for the unesterified species are shown below.

Method of administration:

The cephalosporin derivatives were orally administered in suspensions in water containing 125 mg (potency), calculated as the unesterified species (II).

Assay:

The assay was conducted using Bacillus subtillis as the test organism.

Results:

| Compound | Urinary recovery (%) Hours 0-8 |
|---|---|
| Example 2 | 30.4 |
| Example 4 | 35.7 |
| Example 8 | 29.4 |

Claim

1. A cephalosporin derivative of the general formula:

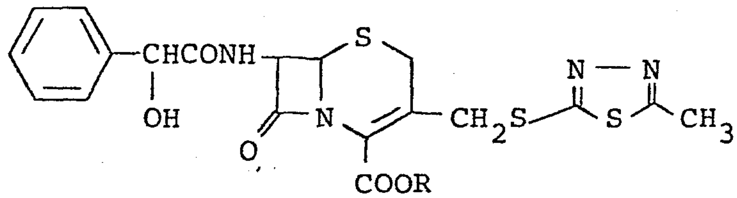

wherein R represents $-CH_2OCOR_a^1$ (wherein $R_a^1$ represents an alkyl group), $-\underset{CH_3}{CH}-OCOR_b^1$ (wherein $R_b^1$ represents an alkyl group), $-\underset{CH_3}{CH}-OCOOR_c^1$ (wherein $R_c^1$ represents an alkyl group),

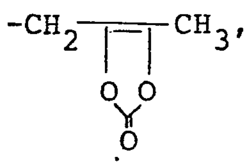, or phthalidyl group.

2. A cephalosporin derivative as claimed in Claim 1, wherein R is 1-acetoxyethyl, 5-methyl-1,3-dioxolen-2-on-4-ylmethyl, 1-isobutyloxyethyl, 1-pivaloyloxyethyl or 1-ethoxycarbonyloxyethyl.

3. The cephalosporin derivative of Claim 1: 1'-acetoxyethyl 7-[D-mandelamido]-3-[(5-methyl-1,3,4-thiadiazol-2-yl)thiomethyl]-3-cephem-4-carboxylate.

4. The cephalosporin derivative of Claim 1: (5-methyl-1,3-dioxolen-2-on-4-ylmethyl)-7-[D-mandelamido]-3-[(5-methyl-1,3,4-thiadiazol-2-yl)thiomethyl]-3-cephem-4-carboxylate.

5. The cephalosporin derivative of Claim 1: 1'-ethoxycarbonyloxyethyl 7-[D-mandelamido]-3-[(5-methyl-1,3,4-thiadiazol-2-yl)thiomethyl]-3-cephem-4-carboxylate.

6. A method for producing a cephalosporin derivative as claimed in Claim 1 which comprises reacting a compound of the formula:

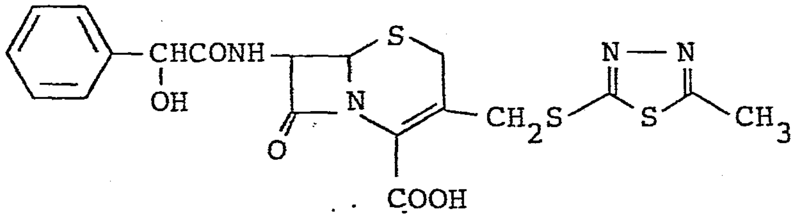

(II)

with a compound of the general formula:

R - OH (III)

wherein R has the same meaning as above, or reacting a compound of the formula:

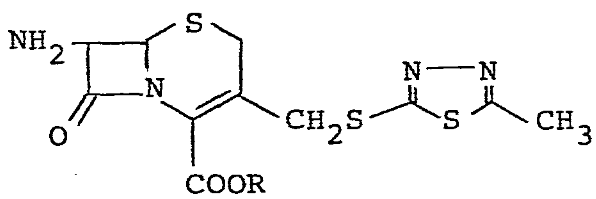

(V)

with a compound of the formula:

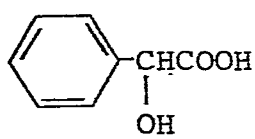

(VI)

0124618

# INTERNATIONAL SEARCH REPORT

International Application No. PCT/JP83/00394

**I. CLASSIFICATION OF SUBJECT MATTER** (if several classification symbols apply, indicate all) [3]

According to International Patent Classification (IPC) or to both National Classification and IPC

Int. Cl.$^3$ C07D 501/36, A61K 31/545

**II. FIELDS SEARCHED**

Minimum Documentation Searched [4]

| Classification System | Classification Symbols |
|---|---|
| I P C | C07D 501/36, A61K 31/545 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| A | JP,B1, 49-45880 (Eli Lilly and Co.), 6. December. 1974 (06. 12. 74) & DT,B, 2018600 | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| January 23, 1984 (23. 01. 84) | February 6, 1984 (06. 02. 84) |
| International Searching Authority [1] | Signature of Authorized Officer [20] |
| Japanese Patent Office | |